# EUROPEAN PATENT APPLICATION

(11) **EP 4 092 128 A1**
(43) Date of publication of application: **23.11.2022**
(21) Application number: 21741052.1
(22) Date of filing: 13.01.2021
(51) Int. Cl.: C12P 21/02, C12P 21/00, C12N 9/10, C12N 9/42, C12N 15/31, C12N 1/15

(54) **FACTOR REGULATING PROTEIN EXPRESSION EFFICIENCY OF TRICHODERMA REESEI, AND REGULATION METHOD AND USE THEREOF**

(30) Priority: 13.01.2020 CN 202010032308
(71) Applicant: CAS Center for Excellence in Molecular Plant Sciences, Shanghai 200032 (CN)
(72) Inventor: ZHOU, Zhihua, Shanghai 200032 (CN); ZOU, Gen, Shanghai 200032 (CN); LIU, Rui, Shanghai 200032 (CN); CHAI, Shunxing, Shanghai 200032 (CN)
(74) Representative: EP&C
(86) International application number: PCT/CN2021/071349
(87) International publication number: WO 2021/143696

(57) **Abstract**

Provided are a factor regulating the protein expression efficiency of *Trichoderma reesei,* and a regulation method and the use thereof. The factor regulating the protein expression efficiency of *Trichoderma reesei* comprises a non-transcriptional regulatory factor that affects the efficiency of protein expression, synthesis or secretion of *Trichoderma reesei.* Further provided are the use thereof in protein expression, synthesis and secretion of *Trichoderma reesei* and constructed recombinant *Trichoderma reesei* strains.

## Description

### FIELD OF THE DISCLOSURE

The present disclosure belongs to the field of biotechnology, and more particularly, to a factor for regulating the protein expression efficiency of *Trichoderma reesei,* a regulation method and use thereof.

### BACKGROUND ART

*Trichoderma reesei* is the most utilized strain in the cellulase industry. *Trichoderma reesei* strain Qm6a is the starting strain of various high-yielding cellulase strain. In order to obtain a new strain with high cellulase production, a more efficient cellulase-yielding mutant strain Qm9414 was obtained from Qm6a through two rounds of linear accelerator mutagenesis and screening, but it can still be repressed by carbon metabolism, as the synthesis of cellulose can be gradually inhibited under increasing glucose concentration over time in fermentation. In order to obtain higher cellulase production, Qm6a was re-cultured, and a high-yielding strain Rut-C30 resistant to carbon metabolism repression was obtained through three rounds of mutagenesis and screening: in the first round, strain M7 was obtained through UV mutagenesis and anti-metabolic repression screening; in the second round, M7 was mutated by N-nitroguanidine to mutant NG14, with increased extracellular protein production and filter paper activity by 1 and 4 times respectively compared with Qm9414; in the third round, a more efficient cellulase-yielding strain Rut-C30 was obtained through ultraviolet mutation and anti-metabolic repression screening on NG14. In addition, another mutant strain RL-P37 was obtained through UV mutagenesis on NG14, and, another high-yielding strain widely used in industry, CL-847, was obtained from mutagenesis on Qm9414. Due to its high yield of cellulase, it is widely used in the fields of textile, papermaking, pulping and bioenergy in industry. At present, the highest fermentation level of the fungus can reach a protein yield of 100 g/L. It has strong protein secretion ability and is safe for humans and animals. It has been developed as a suitable fungal host for expressing homologous and heterologous proteins. In the industrial application of cellulase, the further improvement of the ability of *Trichoderma reesei* in enzyme production and protein secretion is beneficial to its broader prospects in the application market of cellulase.

Although conventional mutagenesis and culturing can obtain a number of desired industrial strains, Kubicek *et al.* believed that from 1978 to 1991, the mutant strains of *Trichoderma reesei* obtained by conventional mutagenesis processes had not shown significant improvement in the ability of enzyme production. In recent years, with the further development of molecular biology, people have gradually shifted from conventional biological processes such as traditional mutagenesis and culturing, development in fermentation conditions, to systematically biological processes using comparative genomics, proteomics, transcriptomics and metabolomics, in order to analyze and understand the functions and related molecular mechanisms of important mutant genes of some high-yielding strains, and use these mechanisms to carry out further genetic modification to obtain productive strains that are more in line with industrial needs. However, given the undiscovered mechanism of induced cellulose production of *Trichoderma reesei* and the unresolved reason why *Trichoderma reesei* mutants have strong protein secretion abilities, *Trichoderma reesei* has not been fundamentally modified to have higher cellulase production and protein secretion capacity.

Therefore, more in-depth engineering of *Trichoderma reesei* is still needed to improve its enzyme production capacity and protein secretion capacity, reduce the industrial production cost of cellulase, and obtain useful industrial strains.

### SUMMARY OF THE DISCLOSURE

Provided herein is a factor for regulating the efficiency of protein expression by *Trichoderma reesei,* a regulation method and use thereof.

In a first aspect of the present disclosure, provided is a method for improving the efficiency of protein expression by *Trichoderma reesei,* comprising: down-regulating a target gene in the genome of *Trichoderma reesei,* wherein the target gene is selected from: a gene encoding ORF4 protein, a gene encoding β-glucosidase.

In a preferred embodiment, the protein is a heterologous protein or an endogenous protein of *Trichoderma reesei.*

In another preferred embodiment, the endogenous protein includes (but is not limited to): cellulase, hemicellulase, β-glucosidase, carbohydrate permease, and enzymes related to protein synthesis and secretion pathways.

In another preferred embodiment, the heterologous protein includes non-cellulase secreted proteins; preferably, the heterologous protein includes (but is not limited to): heterologous glucosidase, heterologous feruloyl esterase FEA, structural proteins (such as spider silk-derived proteins, mulberry silk-derived proteins, etc.), functional proteins (such as immunomodulatory proteins, serum albumin, etc.).

In another preferred embodiment, the gene encoding the heterologous protein is inserted into the genome of *Trichoderma reesei* (as a substitution); or, the gene encoding the heterologous protein is introduced into the *Trichoderma reesei* cell via an expression vector.

In another preferred embodiment, the gene encoding the heterologous protein is inserted into the cbhl site in the genome of *Trichoderma reesei* (as a substitution).

In another preferred embodiment, the starting strain of *Trichoderma reesei* is Rut-C30, Qm9414, or RC30-8.

In another preferred embodiment, down-regulating a target gene in the genome of *Trichoderma reesei* comprises: inhibiting, knocking out, silencing or mutating the target gene, or inhibiting the expression or activity of the protein encoded by the target gene; or transferring a down-regulator that down-regulates the target gene into *Trichoderma reesei* (preferably, the down-regulator including an agent that inhibits or knocks out the target gene or inhibits the expression or activity of the protein encoded by the target gene); or regulating the upstream signaling pathway or upstream gene of the target gene, so that the target gene is down-regulated.

In another preferred embodiment, the target gene is knocked out by using CRISPR/Cas technology for gene editing; or by homologous recombination technology in which the segment where the target gene is located is modified with a deletion or an insertion; or by interfering with the expression of the target gene by using an interfering molecule, wherein the interfering molecule is dsRNA, antisense nucleic acid, small interfering RNA or microRNA, or a construct that can express or form said dsRNA, antisense nucleic acid, small interfering RNA, microRNA, that targets and inhibits or silences the target gene or transcript thereof.

In another preferred embodiment, the ORF4 protein has the amino acid sequence set forth in SEQ ID NO: 2, including homologues (homologous sequences) thereof.

In another preferred embodiment, the β-glucosidase has the amino acid sequence set forth in SEQ ID NO: 15, including homologues (homologous sequences) thereof.

In another preferred embodiment, the gene encoding ORF4 protein is knocked out by introducing a feruloyl esterase FEA expression cassette to homologously replace the gene encoding the ORF4 protein, and at the same time, introduce the feruloyl esterase FEA expression cassette.

In another preferred embodiment, the gene encoding ORF4 protein has the nucleotide sequence set forth in SEQ ID NO: 1, and the gene function can be disrupted by targeting and cleaving at positions 257-277 of said sequence.

In another preferred embodiment, the gene encoding β-glucosidase has the nucleotide sequence set forth in SEQ ID NO: 3, and the gene function can be disrupted by targeting and cleaving at positions 2607-2626 of said sequence.

In another aspect of the present disclosure, provided is the use of a regulator of a target gene for improving the efficiency of protein expression by *Trichoderma reesei;* wherein the regulator includes a down-regulator; wherein the down-regulator targets ORF4 protein or its encoding gene or targets endogenous glucosidase or its encoding gene.

In another preferred embodiment, the down-regulator includes an agent (e.g., an sgRNA shown in SEQ ID NO: 14) that targets and knocks out the target gene via CRISPR/Cas gene editing; or an agent that modifies the segment where the target gene is located with a deletion or an insertion via homologous recombination so that the target gene is knocked out (e.g., an agent targeting the homology arm of the target gene); or an interfering molecule, including dsRNA antisense nucleic acid, small interfering RNA or microRNA, or construct that can express or form the dsRNA, antisense nucleic acid, small interfering RNA or microRNA, that target and inhibit or silence the target gene or transcript thereof.

In another aspect of the present disclosure, provided is a recombinant *Trichoderma reesei* strain or a spore, mycelium or protoplast thereof, in the genome of which a target gene is down-regulated; wherein the down-regulated target gene is selected from: a gene encoding ORF4 protein, a gene encoding endogenous β-glucosidase.

In a preferred embodiment, in the *Trichoderma reesei* strain or a spore, mycelia or protoplast thereof, the target gene is inhibited, knocked out, silenced or mutated, or the expression or activity of the protein encoded by the target gene is inhibited; or the *Trichoderma reesei* strain or a spore, mycelia or protoplast thereof is transformed with a down-regulator of the target gene; preferably, the down-regulator of the target gene includes: an agent that inhibits or knocks out the target gene, or inhibits the expression or activity of the protein encoded by the target gene.

In another preferred embodiment, the *Trichoderma reesei* strain or a spore, mycelia or protoplast thereof further includes a gene encoding a heterologous protein; preferably, the gene encoding the heterologous protein is introduced into the *Trichoderma reesei* cell via an expression vector, or is inserted into the genome of the *Trichoderma reesei* (via homologous substitution), for example (but not limited to), into the cbhl site.

In another aspect of the present disclosure, provided is the use of the *Trichoderma reesei* strain or a spore, mycelium or protoplast thereof described herein for expressing a heterologous protein or an endogenous protein from *Trichoderma reesei.*

In another aspect of the present disclosure, provided is a method for recombinantly expressing a heterologous protein, comprising: inserting a gene encoding a heterologous protein into the genome of a recombinant *Trichoderma reesei* strain or a spore, mycelium or protoplast thereof as described herein; culturing the *Trichoderma reesei* strain or a spore, mycelium or protoplast thereof so that the heterologous protein is expressed; preferably, wherein the gene encoding the heterologous protein is inserted into the cbhl site in the genome of the *Trichoderma reesei* strain or a spore, mycelium or protoplast thereof (via homologous substitution).

In another aspect of the present disclosure, provided is a method for recombinantly expressing an endogenous protein of *Trichoderma reesei,* comprising: culturing the recombinant *Trichoderma reesei* or a spore, mycelium or protoplast thereof described herein, so that the endogenous protein of *Trichoderma reesei* (e.g., cellulase) is recombinantly expressed.

In another aspect of the present disclosure, provided is a kit for protein expression, comprising: a recombinant *Trichoderma reesei* strain or a spore, mycelium or protoplast thereof as described herein; or a down -regulator of the target gene (such as a plasmid for use in knocking out a target gene, or an RNA interference agent), wherein the target gene is selected from: a gene encoding ORF4 protein, a gene encoding endogenous β-glucosidase.

In a preferred embodiment, the endogenous protein includes (but is not limited to): cellulase, hemicellulase, β-glucosidase, carbohydrate permease, and enzymes related to protein synthesis and secretion pathways.

In another preferred embodiment, the heterologous protein includes non-cellulase secreted proteins; preferably, the heterologous protein includes (but is not limited to): heterologous glucosidase, heterologous feruloyl esterase FEA, structural proteins (such as spider silk-derived proteins, mulberry silk-derived proteins, etc.), functional proteins (such as immunomodulatory proteins, serum albumin, etc.).

Other aspects of the disclosure will be apparent to those skilled in the art from the disclosure herein.

### DESCRIPTION OF THE DRAWINGS

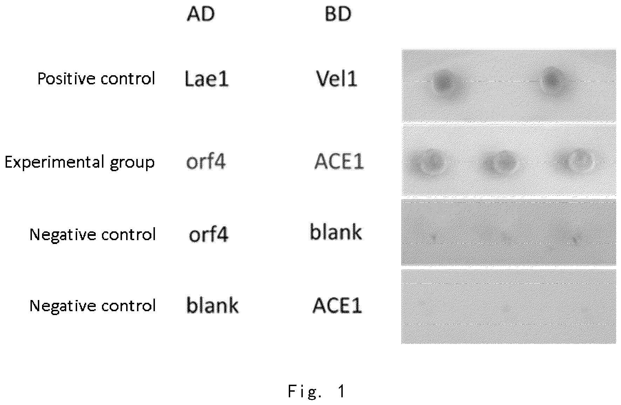

Figure 1. Protein interaction between orf4 and ACE1 is verified using the yeast two-hybrid system. The positive controls are Lae1 and Vel1 which have been reported to have protein interactions; the experimental groups are AD-orf4 and BD-ACE1; and, the negative controls are AD-orf4 and BD empty vector, and AD empty vector and BD-ACE1. The blue color indicates the existence of protein interaction.

### DETAILED DESCRIPTION OF THE DISCLOSURE

*Trichoderma reesei* is a widely used commercial cellulase-yielding microorganism. Although the production of cellulase of common strains currently used in industry has been improved to a certain extent compared with wild-type strains, there is still a certain room for improvement. In addition, the induction mechanism of cellulase production by *Trichoderma reesei* has not been really analyzed. Therefore, it is particularly important to find new key factors related to enzyme production and protein analysis. After long, in-depth research, the inventors found that some target genes in the genome of *Trichoderma reesei* are closely related to the endogenous or exogenous protein production and/or activity of the strain. Down-regulating these target genes can significantly improve the strain's activity and/or production of endogenous or exogenous proteins. Therefore, the target gene or agents and methods for regulating the target gene can be applied to achieve the improvement of Trichoderma reesei, increase the protein expression efficiency of Trichoderma reesei, enhance the production and/or activity of exogenous or endogenous protein from Trichoderma reesei.

As used herein, "ORF4 polypeptide (protein)" refers to a polypeptide having the sequence of SEQ ID NO: 2, including variants or homologues of the sequence of SEQ ID NO: 2 that are isofunctional of the ORF4 polypeptide. These variants include (but are not limited to): deletion, insertion and/or substitution of one or more (e.g., 1-50, preferably 1-30, more preferably 1-20, most preferably 1-10, still more preferably 1-8, 1-5) amino acids, as well as addition or deletion of one or more (usually 20 or less, preferably 10 or less, more preferably 5 or less) amino acids at C-terminal and/or N-terminal.

As used herein, "β-glucosidase" refers to a polypeptide having the sequence of SEQ ID NO: 15, including variants or homologues of the sequence of SEQ ID NO: 15 that are isofunctional of the β-glucosidase polypeptide. These variants include (but are not limited to): deletion, insertion and/or substitution of one or more (e.g., 1-50, preferably 1-30, more preferably 1-20, most preferably 1-10, still more preferably 1- 8, 1-5) amino acids, as well as addition or deletion of one or more (usually 20 or less, preferably 10 or less, more preferably 5 or less) amino acids at C-terminal and/or N-terminal.

Any protein that has high homology with said ORF4 polypeptide or β-glucosidase (for example, a homology of 85% or higher with the sequence set forth in SEQ ID NO: 2 or 15; preferably, a homology of 90% or higher; more preferably, a homology of 95% or higher, e.g., a homology of 98% or 99%), and is isofunctional of ORF4 polypeptide or β-glucosidase is also included in the present disclosure.

Although specific ORF4 polypeptides or β-glucosidase are exemplified in the particular embodiments of the present disclosure, it should be understood that there are some different varieties of *Trichoderma reesei* and the sequences among them are highly conserved, so the homologous polypeptides or polynucleotides derived from these varieties are also encompassed by the present disclosure, and, methods of regulating these homologous polypeptides or polynucleotides (homologues) that are similar or identical to those described herein are also encompassed by the present disclosure. Methods and tools for sequence identity aligning are also well known in the art, such as BLAST.

By gradually knocking out the genes in the genome of *Trichoderma reesei,* the inventors found that the protein secretion capacity of some knockout strains was increased. Single-gene screening of such sequences was carried out, and it was finally found that the methyltransferase in this region was associated to the regulation of cellulase production. It was experimentally verified that the methyltransferase enzyme can regulate the secretion of cellulase by regulating the inhibitory factor ACE1 of cellulase. Genes positioned at other loci are also related to the production of cellulase. The knockout of this methyltransferase can increase the FPU activity of *Trichoderma reesei* Rut-C30 by more than 25%. Therefore, it is a key factor for cellulase production, which can be used for the engineering of industrial strains of *Trichoderma reesei,* and has great application values. Further, the inventors knocked out a gene predicted to be related to β-glucosidase, and found that the protein secretion ability of the knockout strain can be further improved. It was experimentally found that the β-glucosidase has strong hydrolysis ability on sophorose, the strongest inducer of enzyme production by *Trichoderma reesei,* which may lead to a decreased amount of the intracellular inducer, which and the methyltransferase described above are both key factors in the induced production of cellulase.

Therefore, the present disclosure determines for the first time that some target genes in the genome of Trichoderma reesei are closely related to the endogenous or exogenous protein production and/or activity of the strain. Based on these new findings, the present disclosure provides a method for improving the efficiency of protein expression by *Trichoderma reesei,* comprising: down-regulating a target gene in the genome of *Trichoderma reesei,* wherein the target gene that is down-regulated is selected from: a gene encoding ORF4 protein, a gene encoding β-glucosidase.

Once the target gene is determined, it can be modulated (down-regulated or mutated) using a variety of methods well known to those skilled in the art, including but not limited to inhibiting, knocking out, silencing or mutating the target gene, or inhibiting the expression or activity of the protein encoded by the target gene; or transferring a down-regulator of the target gene into *Trichoderma reesei;* or carrying out targeted mutagenesis by using site-directed mutagenesis reagents.

The target gene can be down-regulated in a *Trichoderma reesei* strain using a variety of methods known in the art, including gene silencing, blocking, knockout, suppression, and the like. These methods are all included in the present disclosure.

For example, the target gene in the genome can be modified by a gene blocking technology based on gene insertion or deletion via homologous recombination, so that the target gene is blocked; it is also possible to design interfering RNA or antisense nucleotide for the target gene to inhibit or silence target gene expression.

A method for down-regulating a target gene is gene blocking technology. In a preferred embodiment of the present disclosure, a target gene blocking plasmid is constructed *in vitro,* by which another element is inserted into the chromosomal target gene of the *Trichoderma reesei* strain via homologous recombination, so that the target gene on the chromosome can no longer encode the active protein. When performing gene blocking, the selection of irrelevant elements, such as using some resistance genes, is easily within the skill of those skilled in the art. A method of gene blocking (knockout) can be found, for example, in Genetic Manipulation of Streptomyces: a Laboratory Manual.

When designing a construct for gene blocking or knockout, it is preferable to include a resistance screening gene at the same time, so as to facilitate the subsequent screening of strains engineered with gene blocking or knockout.

The present disclosure also provides a *Trichoderma reesei* strain in which the target gene is down-regulated, or a spore, mycelium or protoplast thereof; more particularly, a *Trichoderma reesei* strain in which the target gene is deleted by using CRISPR/Cas technology, or a spore, mycelium or protoplast thereof, wherein the strain or a spore, mycelium or protoplast thereof does not express the target gene or express at a significantly reduced level. The present disclosure also relates to the use of the strain or a spore, mycelia or protoplast thereof for improving the efficiency of protein expression by *Trichoderma reesei.*

Based on the work of the inventors, the present disclosure also provides a kit for protein expression, comprising: a genetically engineered (recombinant) *Trichoderma reesei* strain, or a spore, mycelium or protoplast thereof as described herein.

The present disclosure also provides a kit for protein expression, comprising: a down-regulator of the target gene, such as a plasmid for use in knocking out a target gene, or an RNA interference agent, or a site-directed mutagenesis reagent and the like.

The kit for protein expression may also include other reagents suitable for the production process of *Trichoderma reesei,* such as the basal medium of *Trichoderma reesei.*

The kit for protein expression may also include instructions for use, explaining the method for culturing the *Trichoderma reesei,* or the method for using the down-regulator or site-directed mutagenesis reagents to down-regulate the target gene by *Trichoderma reesei.*

In the examples of the present disclosure, the inventors found by knocking out the ORF in the genome of Rut-C30 (SEQ ID NO: 1) that this region of sequence is related to the cellulase production and protein secretion ability of *Trichoderma reesei.* Higher production was obtained by expressing a heterologous gene in this region via homologous recombination than expressing at other sites, based on which the production can be further improved by knocking out a gene predicted to be a β-glucosidase gene (SEQ ID NO: 3). Therefore, the genes and non-coding polynucleotide sequences involved in the present disclosure have good industrial application prospects for *Trichoderma reesei* strains.

In a specific embodiment of the present disclosure, after knocking out the polynucleotide sequence of ORF4 in *Trichoderma reesei* Rut-C30 strain, the enzymatic activity of cellulase produced by spores of such modified strain is higher than 15 IU/mL (U/mL); preferably higher than 18 IU/mL.

In another embodiment of the present disclosure, after expressing via homologous recombination the feruloyl esterase FEA expression cassette (SEQ ID NO: 5) at the cbhl site in the *Trichoderma reesei* Rut-C30 strain, and further knocking out the polynucleotide sequence set forth in SEQ ID NO: 1, the enzymatic activity of the cellulase produced by spores of such modified strain is higher than 200 IU/mL; preferably higher than 220 IU/mL.

In another specific embodiment of the present disclosure, the polynucleotide set forth in SEQ ID NO: 3 is further knocked out in a high heterologous β-glucosidase yielding U10 strain in which SEQ ID NO: 1 has been deleted in *Trichoderma reesei,* the enzymatic activity of the β-glucosidase enzyme (pNPGase) produced by spores of such modified strain is higher than 2000 IU/ml; preferably higher than 2500 IU/mL.

High-yielding strains are obtained through the transformation of the present disclosure, and the carbon source of the transformant fermentation culture is simple and easy-to-obtain agricultural wastes such as bran, which has a very obvious effect in improving the hydrolysis efficiency of cellulase preparations, such as food and other industries, with greatly reduced production cost, and wider potential for industrial application.

Further, the active sites described herein can also be used for recombinant expression of heterologous genes. A heterologous gene expression cassette can be introduced into the genome, preferably into the cbhl site, by means of agrobacterium-mediated transformation, PEG-mediated protoplast transformation, technical means based on ku70/ku80, CRSIPR/Cas9, etc., preferably in place of part or all of the original sequence, for recombinant expression. Further improvement can be carried out to obtain derivative strains with higher yield or more optimized enzyme system or other engineered strains of recombinant proteins. Marker-free genetic manipulation can be further established based on these engineered strains, which has great application prospects in industry.

Furthermore, the strain of the present disclosure can be used as the basis for further optimization of derivative strains or engineering strains of other recombinant proteins. The constructed marker-free screening system can be used as the object of molecular manipulation, for genetic manipulations such as knock-in and knock-out of genes. High-yielding engineered strains can be further obtained via targeted genetic modifications, or overexpression of *Trichoderma reesei* cellulase genes with too low expression, such as overexpression of cellulase activator Xyr1, etc., or knockout of cellulase repressor Cre1, etc.

The transformed strain of the present disclosure is a living cell. Once the spores, mycelia, protoplasts and related culture mixtures comprising the living cells of the strain of the present disclosure are obtained, the strains of the present disclosure can be obtained in large quantities by means of inoculation, passage, regeneration, etc. They are usually inoculated into a solid plate medium or a liquid medium for expansion of the strain to obtain the living cells of the present disclosure. The obtained living cells can be further subjected to laboratory domestication, genetic culturing and molecular genetic manipulation to obtain mutants and transformants. The cells of the present disclosure can also be utilized as host cells for heterologous expression.

Methods well known to those skilled in the art can be used to mutagenize the living cells of the present disclosure, resulting in changes in gene coding, enzymatic properties and morphological changes in the living cells. These methods include physical methods such as rays, particles, lasers, and ultraviolet light, and chemical mutagenesis methods such as alkylating agents, base analogs, hydroxylamines, and acridine dyes. Mutagenesis may be multiple generations of one or more of the above methods, and is not limited to these methods. Based on the strains provided by the present disclosure, physical and chemical methods can be further carried out for culturing, and new cellulase genes and related regulatory genes can also be introduced to produce further improved enzyme production performance of the obtained mutants and transformants. The method of culturing can be a combination of one or more of the above.

Those skilled in the art can use the polynucleotide sequences of the present disclosure to construct expression constructs (vectors) and further engineer host cells by using well-known methods, for example, to make further improvement on the signaling, signaling pathway(s), and proteins involved cellulase production that have been discovered or newly discovered in the strain (e.g., to increase the expression of beneficial factors and to reduce the expression of harmful factors).

Transformation of host cells with recombinant DNA can be performed using conventional techniques well known to those skilled in the art. The procedures used are well known in the art, for example but not limit to agrobacterium-mediated fungal transformation, protoplast transformation, electroshock transformation, CRISPR/Cas9 genome editing, biolistic methods, etc.

The optimized strain of *Trichoderma reesei* obtained by the disclosure can utilize the industrial and agricultural production wastes containing lignocellulose, such as bran, corn steep liquor, soybean meal powder and other cheap raw materials, to produce cellulase through liquid or solid state fermentation, and the advantages brought about thereby are that the cellulase produced by the optimized strain has high activity, suitable components and strong ability to hydrolyze lignocellulose. The disclosure provides a new strain resource for solving the problems of high production cost and low saccharification efficiency caused by low enzyme activity and unsuitable enzyme components in the utilization of cellulose resources.

In the present disclosure, the engineered *Trichoderma reesei* strain obtained by introducing a FEA expression cassette and homologously replacing cbhl and further knocking out SEQ ID NO: 1 can utilize industrial and agricultural production wastes containing lignocellulose, such as bran, corn steep liquor, soybean meal powder, to produce cellulase through liquid or solid state fermentation, and the advantages brought about thereby are that the cellulase produced by the engineered strain has high activity, suitable components and strong ability to hydrolyze lignocelluloses, and, the yield of this strain was 2 times that of the randomly introduced FEA expression cassette under the same fermentation conditions. The disclosure provides a new strain resource for solving the problems of high production cost and low saccharification efficiency caused by low enzyme activity and unsuitable enzyme components in the utilization of cellulose resources.

In the present disclosure, the optimized strain of *Trichoderma reesei* obtained by knocking out SEQ ID NO: 3 can utilize the industrial and agricultural production wastes containing lignocellulose, such as bran, corn steep liquor, soybean meal powder and other cheap raw materials, to produce cellulase through liquid or solid state fermentation, and the advantages brought about thereby are that the cellulase produced by the optimized strain has high activity, suitable components and strong ability to hydrolyze lignocellulose. The disclosure provides a new strain resource for solving the problems of high production cost and low saccharification efficiency caused by low enzyme activity and unsuitable enzyme components in the utilization of cellulose resources.

The optimized strain of *Trichoderma reesei* obtained by the method of the disclosure can utilize the industrial and agricultural production wastes containing lignocellulose, such as bean cake flour and other cheap raw materials, to produce cellulase through liquid or solid state fermentation, and the advantage lies in the significantly increased cellulase production. The knockout strains of the present disclosure can provide expression hosts for the expression of non-cellulase secreted proteins.

The use of the present disclosure in preparing cellulase and heterologous proteins can be realized by liquid fermentation. As a preferred embodiment, the fermentation method includes: (1) activating *Trichoderma reesei* species on a slant or flat plate made of potato culture medium, preparing a spore suspension with a concentration of 10 ⁶ to 10 ⁸ mL⁻¹ after activation, and, inoculating Sabouraud's medium (seed medium: 1% yeast extract, 1% peptone, 4% glucose) with 10% inoculum volume, shaking the culture at 28 °C, 200 rpm to obtain a seed liquid, and then inoculating the seed liquid into the liquid fermentation medium with 10% inoculums volume, with the initial pH at 5.0, and the liquid volume of 10 mL in a 50 mL conical flask, shaking at 28 °C, 200rpm shaker for 5-7 days; (2) separating by centrifugation the obtained fermentation broth obtained in step (1), and collecting the supernatant as a crude enzyme liquid; wherein the inducible fermentation medium was an inorganic salt culture medium(0.4% KH₂PO₄, 0.28% (NH₄)₂SO₄, 0.06% MgSO₄7H₂O, 0.05% CaCl₂, 0.06% urea, 0.3% tryptone, 0.1% Tween 80, 0.5% CaCO₃, 0.001% FeSO₄ • 7H₂O, 0.00032% MnSO ₄•H₂O, 0.00028% ZnSO₄•7H₂O, 0.0004% CoCl₂) comprising 5% inducer (3% microcrystalline cellulose and 2% bran).

The culture medium and method for culturing the strain of the present disclosure are not limited to those disclosed above, and other medium and methods conventionally used for culturing *Trichoderma reesei* can also be applied in the present disclosure.

As described above, the fermentation system can be scaled up for industrial production. Depending on the size of the system, those skilled in the art can make appropriate adjustments to facilitate the growth or production of strains based on their general knowledge.

As described above, the crude enzyme liquid of liquid fermentation can produce relatively pure cellulase, enzyme powder or other heterologous recombinant proteins by ultrafiltration, salting out or organic solvent precipitation and other methods. The filter paper activity (FPA), CMC enzyme activity, β-glucosidase enzyme activity and other recombinant protein activities of the produced cellulase are determined after fermentation. It should be understood that the methods of separating and purifying cellulase and recombinant proteins are not limited to those provided in the present disclosure, and other methods known to those skilled in the art can also be applied in the present disclosure.

The present disclosure will be further described below in conjunction with specific examples. It should be understood that these examples are only used to illustrate the present disclosure but not to limit the scope of the present disclosure. The experimental methods that do not indicate specific conditions in the following examples are usually in accordance with conventional conditions such as those described in J. Sambrook et al., Molecular Cloning: A Laboratory Manual, 3rd Edition, Science Press, 2002, or in accordance with the conditions suggested by the manufacturers.

### Example 1 - Acquisition of SEQ ID NO: 1 polynucleotide

### 1. Cultivation of Trichoderma reesei strain

*Trichoderma reesei* Rut-C30 strain (purchased from ATCC, strain number: ATCC 56765) was inoculated on a plate made of potato culture medium, placed at 28°C for 7 days, and a spore suspension at a concentration 10⁶ to 10⁸ spores/mL was prepared, inoculated into Sabouraud's medium (seed medium (v/v): 1% yeast extract, 1% peptone, 4% glucose) at an inoculum volume of 10% (v/v), and incubated at 28°C, 200 rpm shaking, to obtain a liquid culture of the strain.

### 2. Extraction of Trichoderma reesei genomic DNA

The liquid culture was collected into a centrifuge tube, and centrifuged at 12,000 g to obtain mycelia. The supernatant was discarded and into the pellet 0.5 ml phenol:chloroform:isoamyl alcohol (*v*:*v*:*v* =25:24:1) and then 0.5 ml DES (0.1M Tris-HCl, pH 8.0, 1.0% SDS, 2% Triton X-100, 10 mM EDTA, 0.1 M NaCl) was added. After adding 0.1 g beads, the mixture was put in Fastprep, 6 M/S, crushed for 30s and repeated once every 5 minutes. The lysed liquid was centrifuged at 12,000 g for 10 min at 4°C. 0.4ml of supernatant was aspirated, and into which isopropanol of an equal volume was added. The mixture was precipitated at -20 °C for 30 minutes and centrifuged at 12000g, 4°C, for 10min. The supernatant was discarded. After the precipitate was rinsed with 75% ethanol, centrifuged at 12,000 g at 4°C for 5 min. The supernatant was discarded, and the pellet was air-dried for 5 min, and then 200 µl of sterile water was added.

### 3. Cloning of orf4 polynucleotide (SEQ ID NO: 1)

The *Trichoderma reesei* genomic DNA extracted above was used as a templete, using a forward primer: 5' ATGTCCCAATACGACTCCATCGG 3' (SEQ ID NO: 6) with an XbaI recognition site: TCTAGA added to its 5' end, and a reverse primer: 5' TTACAACCGCAACTAAAACAC 3' (SEQ ID NO: 7), with an XbaI recognition site added to its 5' end.

The PCR product was purified and digested with XbaI, and the digested DNA fragment was recovered using the Axygen PCR product column recovery kit (with Kan and hyg screening markers, purchased from Sigma). After dephosphorylation treatment, this DNA fragment and the vector pHDt/sk that was subjected to the same enzymatic disgestion and recovered were ligated with T4 DNA ligase overnight at 16°C to obtain vector pHDt/sk-full. The sequence was verified by sequencing.
SEQ ID NO: 1 (*orf4*) nucleic acid sequence: 
ATGTCCCAATACGACTCCATCGGCGCCTCCTATAATGTCCTTGAGCAACTTGCCTACAGAG
CTGTGGAGAAGTACAACGTGTACACAACTATTAAGCCACTACTCAGACCGGGGGTGCATG
TTCTAGATCTTGCCTGCGGTACCGGCTTCTACTCATCACATCTTCTTGCATGGGGCGCTGA
 
AGACGTCCTCGGCGTGGATATATCATCTGCAATGCTTGAGAGTGCCGTATCTCGATTATCA
TCAGAGATTTCTGCGGGAAAGGCACAATTCGTGTTGGGGATGGTGTCATTCCACAGTC
GTTCGCCCCAGACAACAGTCGAGGATTTTCGGCATGGTCTTTGGTGCGTGGTTTCTCAAT
TATGCGTCGAGCAAAGACCAATTGGTTGCCATGTTCACAAATATCTCCCTCAACCTCACAG
TGGATGGCGTTTTTGTGGGAGTTGTCCCTTATCCAACAAATGATATCCAGGAACGGGCCA
AAAACTCTGAGCAAATCTCACTACGGCAGTATTTCCCACGCAACGAGTATGTAGAAGAGC
TGAGCTCAGGCGACGGCTGGAGCTTACGGGTATTTCAAGTGATGATGGGGTGGATTTCAT
GACCTGGCATATGAAGAAAAGCGTTTACGAGGAAGCCGCAAGACTGGGAGGTTTGCGGG
GCAAATTGGAGTGGCGCGATGAGACTCTGGGAACAGAAAATTCAGAGCAGCAATTTGGTT
TGACAGCTGACGAGTGGTCAATCAGAGTACAAAACCCTCACTTGGGAATATTGGTCGCCT
GGAAGAGTTGGTTCAATACTGGAAAGAGAACAAATCGTTAAGCTGAGAGTAGTAACAGG
AACGGAGGCTATGGAGCATCTTCATAATACCTTTGGCAGTTAACAAACGTCATCAATTCAT
AACTGAGCAACTATATACCTGATACTAGACTGGTTATCATGGTATCTTAGGAGAGGGGTT
GAAGAAGGTAAGTGTTTTAGTTGCGGTTGTAA
SEQ ID NO: 2 (*orf4*) polypeptide sequence:

MSQYDSIGASYNVLEQLAYRAVEKYNVYTTIKPLLRPGVHVLDLACGTGFYSSHLLAWGAEDVLG
VDISSAMLESAVSRLSSEISAGKAQFVLGDGVIPQSFAPDNSRGFFGMVFGAWFLNYASSKDQLVA
MFTNISLNLTVDGVFVGVVPYPTNDIQERAKNSEQISLRQYFPRNELVIMLRL

### Example 2 - Study the function of orf4

### 1. Construction of Trichoderma reesei CRISPR/Cas9 chassis cells

By analyzing the codons of the Cas9-encoding gene of *Streptococcus pyogenes,* using the artificially optimized Cas9 gene (SEQ ID NO: 4; segment from positions 4117 to 4137 is a nuclear localization signal) as a template, and primers Cas9F (ATGGACAAGAAGTACAGCATTGG (SEQ ID NO: 8)) and Cas9R (TTAGACCTTGCGCTTCTTCTTGGG (SEQ ID NO: 9)), the sequence was isolated and obtained by PCR. Using the pDHt/sk vector as the backbone, a codon-optimized Cas9 gene expression vector was constructed.

Ppdc as an optimized promoter for Cas9 gene expression was obtained by amplification using *Trichoderma reesei* genomic DNA as a template, as well as forward primer: 5' ACGACGGCCAGTGC CAAGCTTAGGACTTCCAGGGCTACTTG 3' (SEQ ID NO: 10) and reverse primer: 5' GATTGTGCTGTAGCTGCGCTGCTTTGATCGTTTTGAGGTGC 3' (SEQ ID NO: 11).

Tpdc as an optimized terminator was obtained by amplification using *Trichoderma reesei* genomic DNA as a template, as well as forward primer 5' AGAAGAAGGGAA GGTGTGACCCGGCATGAAGTCTGACCG 3' (SEQ ID NO: 16) and reverse primer 5' TAATTGCGCGG ATCCTCTAGATGGACGCCTCGATGTCTTCC 3' (SEQ ID NO: 17).

The vector was constructed by one-step cloning process. The recombinant vector pDHt/sk-ppdc-Cas9-tpdc containing the inducible promoter was successfully constructed by using the verified sequence.

The plasmid constructed above was transformed into *Agrobacterium tumefaciens* AGL1, then transformed into *Trichoderma reesei* Rut-C30 under the mediation of *Agrobacterium tumefaciens,* with hygromycin as the screening marker. The grown transformants were inoculated in the SDB culture medium, and DNA was extracted after 2 days. Positive transformants were identified using the extracted DNA as a template, a forward primer: 5' TACGTCGGCCCCCTGGCC 3' (SEQ ID NO: 12) designed for the promoter on the vector, and a reverse primer of the Cas9 gene: 5' GAGGTTGTCAAACTTGCGCTGCG 3' (SEQ ID NO: 13). The obtained transformants were named C30-pc, respectively.

### 2. Functional knockout of orf4

A sgRNA was established: 5' TAATACGACTCACTATA **GGGAAAGGCACAATTCGT GT** GTTTTAGAGCTAGAAATAGC 3' (SEQ ID NO: 14) targeting SEQ ID NO: 1 (cleaved at positions 257-277 of the gene to disrupt gene function).

The constructed sgRNA was ligated into pMD18T (purchased from Takara, Amp-resistant), which was used as a template. The obtained in vitro transcription template was extracted and purified with phenol:chloroform:isoamyl alcohol (25:24:1), and subjected to DNA dissolution using nuclease free water, ready for *in vitro* transcription of gRNA.

For *in vitro* transcription of gRNA, reference was made to the kit instructions for specific procedures. C30-pc spores were inoculated on PDA plates, cultured at 28 °C for 7 days, and the spores were washed and removed with 0.85% NaCl+0.02% Tween 80. A piece of cellophane was covered on the PDA plate and flatten with sterilized tweezers, onto which about 150ul of spore suspension was applied, spread evenly with a glass rod, with the same procedure repeated for 6 times, and incubated at 28°C for about 14 hrs. They were treated with lyase (Sigma#L1412 lyase) to break the cell walls. The protoplast suspension was obtained after removing cell wall debris, mixed with purified gRNA fragments, and transformed by PEG-mediated protoplast transformation. They were spread on a screening plate, and incubated at 28°C. The growth of mycelium can be observed in about 5 days. After the transformant grew out, the knockouts with corresponding sgRNAs can be obtained via verification.

### 3. Enzyme activity screening for knockouts

*(1) Trichoderma reesei* strains were inoculated on a plate made of potato culture medium and placed at 28°C for 7 days to prepare a spore suspension with a concentration of 10⁶ to 10⁸ spores/mL. The suspension was inoculated at an inoculum volume 10%(v/v) into Sandburg's medium (seed medium (v/v): 1% yeast extract, 1% peptone, 4% glucose), incubated at 28°C under 200 rpm shaking, to obtain a seed liquid. Then the seed liquid was inoculated into a liquid fermentation medium with 10% (v/v) inoculum volume. The initial pH was 5.0. 10 mL of liquid was contained in a 50mL conical flask, and cultured in a shaker at 28°C and 200rpm for 5-7 days;
(2) The fermentation liquid obtained in step (1) was centrifuged, and supernatant was taken as crude enzyme liquid; the fermentation medium was an inorganic salt culture medium (0.4% KH₂PO₄, 0.28% (NH₄)₂SO₄, 0.06% MgSO₄•7H₂O, 0.05% CaCl₂, 0.06% urea, 0.3% peptone, 0.1% Tween 80, 0.5% CaCO₃, 0.001% FeSO₄•7H₂O, 0.00032% MnSO₄•H₂O, 0.00028% ZnSO₄•7H₂O, 0.0004% CoCl₂) containing 5% inducer (3% microcrystalline cellulose and 2% bran).
(3) Into 0.05mol/L acetic acid - sodium acetate or citric acid - sodium citrate buffering solution (pH 5.0), *Trichoderma reesei* strain and its knockout crude enzyme solution was added to filter paper, and subjected to hydrolysis at 50-60 °C for 60 min. The enzymatic activity of cellulase on the filter paper was determined (unit: Filter Paper Unit, FPU). The enzyme activities were measured as shown in Table 1.

**Table 1. Filter paper activity of Trichoderma reesei transformants and their knockout strains**

| Strain | C30-pc | Δ 4-1 | Δ 4-2 | Δ 4-3 | Δ 4-4 | Δ 4-5 |
|---|---|---|---|---|---|---|
| FPU(U/ml) | 13.787 | 16.491 | 18.154 | 16.216 | 16.289 | 17.909 |
| SD | 0.145 | 0.503 | 2.218 | 1.721 | 1.422 | 1.876 |

It can be seen from the results in Table 1 that the knockout of *orf4* significantly increased the enzymatic activity of cellulase, so it is associated with the production of cellulase and is a key gene.

### Example 3. Screening of interacting proteins of proteins encoded by SEQ ID NO: 1

Yeast two-hybrid vectors pGADT7 AD Vector and pGBKT7 DNA-BD Vector (purchased from Takara Company) were used to construct orf4 (i.e., SEQ ID NO: 1) and other known transcriptional regulators including: ACE1 (GenBank accession number AF190793), Vel1 (GenBank Accession No. XM 006966084), Hda1 (GenBank Accession No. XP_006968036), Cre1 (GenBank Accession No. AAB01677), Lae1 (GenBank Accession No. AFX86442, etc., to obtain AD-orf4, AD-ACE1, AD-Vel1, AD-Hda1, AD-Cre1, AD-Lae1, AD-Cre1 and BD-orf4, BD-ACE1, BD-Vib1, BD-Hda1, BD-Cre1, BD-Lae1, BD-Cre1, etc. The AH109 yeast strains (purchased from Takara) transformed with different combinations of AD and BD vectors were prepared, using respective blank controls, and were spread on screening plates containing SD+Dropout+Ade+His medium. After culturing at 30°C for 2-3 days, a few colonies were picked to SD+Dropout+3-AT+X-α-GAL nutrient deficiency medium, and cultured at 30 °C for 2-3 days. Two proteins were identified to interact when yeast could grow and turn blue at the same time.

The results showed that the transformants co-transformed with AD-orf4 and BD-ACE1 could grow and turn blue, as shown in Figure 1, indicating that orf4 and ACE1 interact.

ACE1 is a cellulase inhibitor, and the results suggest that orf4 modifies certain sites of ACE1 to activate its function of inhibiting cellulase transcription.

### Example 4. Expression of heterologous protein using orf4

Taking the C30-pc strain obtained in Example 2 as the starting strain, the feruloyl esterase FEA (nucleotide set forth in SEQ ID NO: 5) was homologously substituted for the *Trichoderma reesei* cbhl site using CRISPR/Cas9 technology. Then SEQ ID NO: 1 was knocked out, and the obtained transformants were named U10FEA1 to U10FEA5, with the strains having only homologous replacement at the position of *Trichoderma reesei* cbhl without knocking out SEQ ID NO: 1 used as controls (FEAΔ1). The transformants and C30-pc were inoculated on a plate made of potato medium and cultured at 28 °C for 7 days to prepare a spore suspension with a concentration of 10⁶ to 10⁸ spores/mL. The suspension was inoculated at a inoculum volume of 10% (v/v) into Sandburg's medium (seed medium (v/v): 1% yeast extract, 1% peptone, 4% glucose), incubated at 28 °C, under 200 rpm shaking to obtain seed liquid. Then the seed liquid was inoculated at 10% (v/v) inoculum volume into the liquid fermentation medium. The initial pH was 5.0. 10 mL of the liquid was contained in a 50mL conical flask, and cultured for 5-7 days at 28°C, on 200rpm shaker; the obtained fermentation broth was centrifuged, and the supernatant was taken as crude enzyme liquid; the fermentation medium was an inorganic salt culture medium (0.4% KH₂PO₄, 0.28% (NH₄)₂SO₄, 0.06% MgSO₄•7H₂O, 0.05% CaCl₂, 0.06%urea, 0.3% peptone, 0.1%Tween80, 0.5%CaCO₃, 0.001%FeSO₄•7H₂O, 0.00032% MnSO₄•H₂O, 0.00028% ZnSO₄•7H₂O, 0.0004% CoCl₂) containing 5% inducer (3% microcrystalline cellulose and 2% bran). The fermentation broth was diluted with 0.05 mol/L acetic acid - sodium acetate buffer at pH 6.0, and 50ul of which was added to 0.625mM pNPF (first prepared with DMSO into a 10 nM stock solution, from which 0.5 mL was taken and dissolved in 7.5 mL of 0.05mol/L acetic acid - sodium acetate buffer containing 5% Triton X-100, pH 6.0; for the preparation of pNPF, reference was made to the preparation process described in Analytical Biochemistry 387 (2009) 128 -129), hydrolyzed at 37°C for 10 min, centrifuged, and the supernatant was harvested and read at OD405. The measured feruloyl esterase FEA enzyme activity was shown in Table 2.

**Table 2. pNPF enzymatic activity of C30-pc and its transformants**

| Strain | C30-pc | FEAΔ1 (cbhl replaced) | U10FEA1 (cbhl replaced + orf4 knocked out) | U10FEA2 (rcbhl replaced + orf4 knocked out) | U10FEA3 (rcbhl replaced + orf4 knocked out) | U10FEA4 (rcbhl replaced + orf4 knocked out) | U10FEA5 (rcbhl replaced + orf4 knocked out) |
|---|---|---|---|---|---|---|---|
| pNPF (U/ml) | - | 182.36 | 215.08 | 226.32 | 210.36 | 220.39 | 243.13 |
| SD | - | 12.63 | 15.63 | 12.63 | 17.21 | 15.75 | 25.96 |

It can be seen from the results in Table 2 that upon replacing cbhl and knocking out orf4 (SEQ ID NO: 1), a higher expression of heterologous protein was obtained than only replacing cbhl. Knockout of orf4 is a preferred strategy to increase the expression and yield of heterologous proteins.

### Example 5. Increasing the level of extracellular secreted proteins by manipulating endogenous β-glucosidase

Using *Trichoderma reesei* strain Qm9414 (purchased from ATCC 26921) as the starting strain, the β-glucosidase encoding gene (nucleotide sequence set forth in SEQ ID NO: 3) was knocked out by the method described in Example 1. sgRNA was designed to be: 5' TAATACGACTCACTATA**GGCAAGTATCCGTATCCCGAGTTTTA**GAGCTAGAAATA GC 3' (SEQ ID NO: 19). The nucleotides 2607-2626 set forth in SEQ ID NO: 3 were cleaved to destroy the function of the gene to obtain transformants Δbgl1 to Δbgl3.
Gene of β-glucosidase (SEQ ID NO: 3): 
atgacctcgtttcacgacggcgtcaagttgtccactgtgacgtgtgtcctgtccggcttggtggctctaggaagtgccggacctactgctgcgtcggcaaatgcacaagta
gcggcggctgcggctgcccaagcctgggttccagatggctactatgtgccgccgtattatcctgcgccgtacggtggatgggtggaggattggcaagagagttatacca
aggccaaggctctggtcgattccatgactctggcagaaaagaccaatatcactgcaggaactggcatctatatgggtgagtggtggcttccttgaggatgagaggcagttt
cagactgtgtgtggatgagcgagtaattgtatgctaaccagcgagaacagggtgagttatttggagccttgccaacgtacagaccgaagcctcttcaagggatgaaagg
cttttgtcagaacttcagcaaacgggtcttgtacaccatatgctgggatgatgataacccattgagagattataaactaacactgatcaaggcgatgtgccggaaacacag
gctctgcgtttcgagtttcctttccccagctgtgtctcaacgacagcccggcgggtgtccgacatgcggacaatgtaacagcttttcctgacggaatcaccgtcggcgcaa
cgttcgacaaggccctcatgtacaagcgcggcgtggccattggaaaggagaaccgcggaaagggcgtcaatgtctggctgggaccaactgttgggccgataggccgc
aagccaaagggcggccgcaactgggaaggcttcggtgctgatcctgtgctccaggccgtcggtgctcgagagacgatcaagggcgttcaagagcaggggggtcattgc
gactatcaaacactttatcggcaacgagcaggagatgtaccgcatgtacaatccctttcaatacgcatacagctcaaatattggtacgtgtttccagcggaatgaagagtaa
aagtgagataagatgctgacaatgagttgacgggtaaaagacgataggacgctgcatgaagtatatgcctggccattcgccgagggcatccgagcgggtgttggcgcc
gtcatgatggcttacaatgctgtaagtgattctgagacgagtggcttgattgtccagcagctaattgcttttctcccgccaggtgaacgggactgcgtgctctcaacacccat
atcttatgagtgcccttctcaaggatgagatgggctttcaaggcttcatcatgaccgactggctcgctcacatgtctggcgtcgcttctgccattgccggcctggatatggac
atgcctggcgacgttcagattcccttcttcggcggctcatactggatgtatgagctcactcggtctgctcttaatggttcggttcccatggatcgcatcaacgacgcggctac
acgcatagccgctgcgtggtacaagatgggccaggacaagggattccccgcgacgaatttcgacaccaactctcgtgctgccttcaacccgctgtatcctgccgcgctg
ccactttcgccatttggcatcacaaacgagtttgtaccggtccaagacgatcacgacgtaattgctcgccaaatttctcaggaagcaatcaccctgttgaagaacgacggc
gacatcctccctctatcgccttcgcagcacctcaaggttttcggcaccgatgctcagaaaaacccagacggcatcaattcatgcacagatcgcaattgcaacaagggaac
tctgggccaaggatggggctcgggaactgtcgattacccctatttggacgatcccatctcagctatcactgcagaggccgacaatgtcacgttttacaacacggacaagtt
cccttctgtcggcgaagtatcagatagcgacgtcgccatcgtctttgtcaactcggatgctggcgaaaacacctacaccgtggaaggcaaccacggcgaccgtgacaaa
tccggcttgtacgcatggcacgacggcgataagcttgtgcaggacgccgcgagcaagttcagcaacgtcattgtcgtgatacacactgttggcccccctgatcctcgaga
agtggatcgatctgccatccgtcaaggcggtgctcgtcgcacatctgcccggccaggaagccggcaagtctcttacaaacgtcctctttggacacgcctcgccgtgcgg
acacttgccttattccatcacgaaagaggaggacgaccttcccaaaagcgtcactacgctcattgactcggagttcctcaaccagccccaagacacatacacagagggtc
tgtacattgactaccgctggctcaacaagaacaagaccaagcctcgctacgcctttggccacggtctgagctacacaaacttcaccttcaaggcagcatccatcaaacag
gtcgccaggctgagcgcatacccgccggctcgcccagccaagggcagcacccccgattttgcgcaatccattccatccgcaagcgaagccgttgcgccgtcaggcttc
ggcaagatcccccgctacatctactcttggctgtcgcagggcgatgccaaccgggccatttcagacggcaagacgggcaagtatccgtatcccgacggctactctacc
acccagaagcccggcgctcgcgctggcggcggcgagggaggcaaccccgcgctctgggacgttgcgtacagcctcacggtgacggtgcagaatacgggcgatga
gtatgctggcaaggcctccgtgcaggcatatctccaattcccggacgatatcgactacgatacgccaatcattcagctccgtgactttgaaaagacaaaggagctaaagc
 
cggggggagacgacgactgtgacgctgactcttacacgcaaggacgtcagtgtgtgggacgtggtggcgcaggattggaaggttcctgcggttgacggagggtataag
gtgtggattggggatgccagtgattcgttaagtattgtatgtcatacggatacgttggagtgtgagactggtgttgttgggcctgtgtag
Protein of β-glucosidase (SEQ ID NO: 15):

MTSFHDGVKLSTVTCVLSGLVALGSAGPTAASANAQVAAAAAAQAWVPDGYYVPPYYPAPYGGWVEDWQ
ESYTKAKALVDSMTLAEKTNITAGTGIYMGERCAGNTGSAFRVSFPQLCLNDSPAGVRHADNVTAFPDGITV
GATFDKALMYKRGVAIGKENRGKGVNVWLGPTVGPIGRKPKGGRNWEGFGADPVLQAVGARETIKGVQEQ
GVIATIKHFIGNEQEMYRMYNPFQYAYSSNIDDRTLHEVYAWPFAEGIRAGVGAVMMAYNAVNGTACSQHP
YLMSALLKDEMGFQGFIMTDWLAHMSGVASAIAGLDMDMPGDVQIPFFGGSYWMYELTRSALNGSVPMDRI
NDAATRIAAAWYKMGQDKGFPATNFDTNSRAAFNPLYPAALPLSPFGITNEFVPVQDDHDVIARQISQEAITL
LKNDGDILPLSPSQHLKVFGTDAQKNPDGINSCTDRNCNKGTLGQGWGSGTVDYPYLDDPISAITAEADNVTF
YNTDKFPSVGEVSDSDVAIVFVNSDAGENTYTVEGNHGDRDKSGLYAWHDGDKLVQDAASKFSNVIVVIHT
VGPLILEKWIDLPSVKAVLVAHLPGQEAGKSLTNVLFGHASPCGHLPYSITKEEDDLPKSVTTLIDSEFLNQPQ
DTYTEGLYIDYRWLNKNKTKPRYAFGHGLSYTNFTFKAASIKQVARLSAYPPARPAKGSTPDFAQSIPSASEA
VAPSGFGKIPRYIYSWLSQGDANRAISDGKTGKYPYPDGYSTTQKPGARAGGGEGGNPALWDVAYSLTVTVQ
NTGDEYAGKASVQAYLQFPDDIDYDTPIIQLRDFEKTKELKPGETTTVTLTLTRKDVSVWDVVAQDWKVPAV
DGGYKVWIGDASDSLSIVCHTDTLECETGVVGPV

The transformants and Qm9414 were inoculated onto a plate made of potato medium and cultured at 28°C for 7 days to prepare a spore suspension with a concentration of 10⁶ to 10⁸ spores/mL. The spore suspension was inoculated into Sandburg's medium (seed medium (v/v): 1% yeast extract, 1% peptone, 4% glucose) at a inoculum volume of 10% (v/v), and incubated at 28°C under 200 rpm shaking to obtain a seed liquid. The seed liquid was then inoculated at an inculum volume of 10% (v/v) into the liquid fermentation medium. The initial pH was 5.0. 10 mL of the culture was contained in a 50mL conical flask, and cultured at 28°C, 200rpm in a shaker for 5 to 7 days. The obtained fermentation broth was centrifuged and separated, and the supernatant was collected as crude enzyme liquid; the fermentation medium was an inorganic salt culture medium (0.4% KH₂PO₄, 0.28% (NH₄)₂SO₄, 0.06% MgSO₄•7H₂O, 0.05% CaCl₂, 0.06%urea, 0.3% peptone, 0.1%Tween80, 0.5%CaCO₃, 0.001%FeSO₄•7H₂O, 0.00032% MnSO₄•H₂O, 0.00028% ZnSO₄•7H₂O, 0.0004% CoCl₂) comprising 5% inducer (3% microcrystalline cellulose and 2% bran). The enzyme activity detection is specifically shown in Table 3.

**Table 3. Cellulase enzyme activity of the fermentation broth of Qm9414 and its transformants**

| Strain | Qm9414 | Δbgl1 | Δbgl2 | Δbgl3 |
|---|---|---|---|---|
| FPA(U/ml) | 5.32 | 7.65 | 8.13 | 7.69 |
| SD | 0.87 | 0.58 | 0.57 | 0.91 |

It can be seen from the results in Table 3 that the knockout of endogenous β-glucosidase can significantly increase the production of endogenous cellulase.

### Example 6. Further increased yield of exogenous protein through the manipulation of endogenous β-glucosidase

Using the *Trichoderma reesei* strain (CN Patent No.: 201210258194.6, taking RC30-8 as the starting strain) in which exogenous β-glucosidase *trbgls* is overexpressed, the SEQ ID NO: 1 sequence was knocked out by the method described in Example 2, to obtain U10. Taking U10 as the starting strain, the nucleotide shown in the endogenous β-glucosidase encoding gene SEQ ID NO: 3 was knocked out (sgRNA: 5' TAATACGACTCACTATA**GGCAAGTATCCGTATCCCGA**GTTT TAGAGCTAGAAATAGC 3' (SEQ ID NO: 18); cutting position: positions 2607-2626 of the nucleotide sequence set forth in SEQ ID NO: 3), was knocked out, to obtain a strain the protein function of which was destroyed, and the obtained transformant was named U10ΔB1.

The transformants and U10 were inoculated onto a plate made of potato medium and cultured at 28 °C for 7 days to prepare a spore suspension with a concentration of 10⁶ to 10⁸ spores/mL. The spore suspension was inoculated at an inoculum volume of 10% (v/v) into Sandburg's medium (seed medium (v/v): 1% yeast extract, 1% peptone, 4% glucose), and incubated at 28 °C, under 200 rpm shaking to obtain a seed liquid. The seed liquid was inoculated at an inoculum volume of 10% (v/v) in the liquid fermentation medium. The initial pH was 5.0. 10mL of the culture was contained in a 50mL conical flask, and cultured in a shaker at 28 °C and 200rpm for 5 to 7 days. The obtained fermentation broth was centrifuged and separated, and the supernatant was collected as crude enzyme liquid; the fermentation medium was an inorganic salt culture medium (0.4% KH₂PO₄, 0.28% (NH₄)₂SO₄, 0.06% MgSO₄•7H₂O, 0.05% CaCl₂, 0.06% urea, 0.3% peptone, 0.1% Tween 80, 0.5% CaCO₃, 0.001% FeSO₄•7H₂O, 0.00032% MnSO₄•H₂O, 0.00028% ZnSO₄•7H₂O, 0.0004% CoCl₂) comprising 5% inducer (3% microcrystalline cellulose and 2% bran). Enzyme activity detection is as follows:
The enzymatic activity and physicochemical properties of heterologous glucosidase were detected using p-nitrophenyl-β-D-glucoside (*p*NPG) as a substrate. The specific procedures were as follows:

### (1) Preparation of standard curve for p-nitrophenol (pNP)

PCR plates in 8^{∗}3 array was arranged, into which solutions were added according to Table 4, in a total of 8 groups, and each group was performed in triplicate.

**Table 4**

| | | | | | | | | |
|---|---|---|---|---|---|---|---|---|
| Standard sample No. | 0 | 1 | 2 | 3 | 4 | 5 | 6 | 7 |
| Total amount of pNP (µg) | 0 | 1 | 2 | 3 | 4 | 5 | 6 | 7 |
| pNP volume (µl) | 0 | 1 | 2 | 3 | 4 | 5 | 6 | 7 |
| Supplementary buffer (µl) | 100 | 99 | 98 | 97 | 96 | 95 | 94 | 93 |
| 1M NaCO₃ | 100ul per tube | | | | | | | |

The pNP concentration was 10 mg/ml. 100 µl of 1M NaCO₃ was added to each standard sample as shown in the above table. The reaction was stopped and subjected to developing. 100 µl of sample from each tube was pipetted into an ELISA plate, and the absorbance at 405 nm was measure with a microplate reader. The standard sample number 0 is a blank control. A standard curve was drawn by subtracting the blank value from each sample value.

### (2) Standard enzyme activity assay of heterologous glucosidase

In the 100 µl reaction system, 50 µl of *p*NPG at a concentration of 4 mM was added, and then 50 µl of the enzyme solution diluted to a certain dilution with a 0.1 M NaAc/HAc (pH 5.0) buffer was added. The mixture was allowed to react at 60°C for 10 minutes, and then 100 µl of 1M NaCO₃ was added to terminate the reaction for color development (for the control, 100 µl of 1 M NaCO₃ was added to the above reaction system and then the enzyme solution was added), and the absorbance value at 405 nm was measured with a microplate reader. The enzyme activity unit (U) was calculated using the standard curve by subtracting the control value from the measured value of the sample. The results are shown in Table 5.

Definition of enzymatic activity unit (U): 1 U is the amount of enzyme required to catalyze *p*NPG to produce 1 µmol *p*NP per minute.

**Table 5. The pNPG enzyme activity of the fermentation broth of U10 and its transformants**

| Strain | RC30-8 | U10 | U10ΔB1 | U10ΔB2 | U10ΔB3 | U10ΔB4 | U10ΔB5 |
|---|---|---|---|---|---|---|---|
| pNPG(U/ml) | 6.98 | 1669.39 | 2103.98 | 2286.54 | 2189.36 | 2209.63 | 2503.36 |
| SD | 0.32 | 23.69 | 132.28 | 65.98 | 58.69 | 110.36 | 157.64 |

It can be seen from the results in Table 5 that the knockout of endogenous β-glucosidase can further improve the production of exogenous protein (heterologous glucosidase).

All documents mentioned herein are incorporated by reference in this application as if each document were individually incorporated by reference. In addition, it should be understood that after reading the above teaching content of the present disclosure, those skilled in the art can make various changes or modifications to the present disclosure, and these equivalent forms also fall within the scope defined by the appended claims of the present application.

## Claims

1. A method for improving the efficiency of protein expression by *Trichoderma reesei,* comprising: down-regulating a target gene in the genome of *Trichoderma reesei,* wherein the target gene is selected from: a gene encoding ORF4 protein, a gene encoding β-glucosidase.

2. The method according to claim 1, wherein the protein is a heterologous protein or an endogenous protein of *Trichoderma reesei.*

3. The method according to claim 2, wherein the endogenous protein includes: cellulase, hemicellulase, β-glucosidase, carbohydrate permease, and enzymes related to protein synthesis and secretion pathways; or, the heterologous protein includes non-cellulase secreted proteins; preferably, the heterologous protein includes: heterologous glucosidase, heterologous feruloyl esterase FEA, structural proteins, and functional proteins.

4. The method according to claim 2, wherein the gene coding the heterologous protein is inserted in the genome of *Trichoderma reesei;* or, the gene encoding the heterologous protein is introduced into the *Trichoderma reesei* cell via an expression vector.

5. The method according to claim 1, wherein the gene encoding the heterologous protein is inserted into the cbhl site in the genome of *Trichoderma reesei.*

6. The method according to claim 1, wherein down-regulating a target gene in the genome of *Trichoderma reesei* comprises: inhibiting, knocking out, silencing or mutating the target gene, or inhibiting the expression or activity of the protein encoded by the target gene; or transferring a down-regulator that down-regulates the target gene into *Trichoderma reesei;* or regulating the upstream signaling pathway or upstream gene of the target gene, so that the target gene is down-regulated.

7. The method according to claim 6, wherein the target gene is knocked out by using CRISPR/Cas technology for gene editing; or by homologous recombination technology in which the segment where the target gene is located is modified with a deletion or an insertion; or by interfering with the expression of the target gene by using an interfering molecule wherein the interfering molecule is a dsRNA, an antisense nucleic acid, a small interfering RNA or an microRNA, or a construct that can express or form the dsRNA, antisense nucleic acid, small interfering RNA, or microRNA that targets and inhibits or silences the target gene or transcript thereof.

8. The method according to claim 1, wherein the gene encoding ORF4 protein is knocked out by introducing a feruloyl esterase FEA expression cassette to homologously replace the gene encoding the ORF4 protein, and at the same time, introduce the feruloyl esterase FEA expression cassette; or the gene encoding ORF4 protein has a nucleotide sequence set forth in SEQ ID NO: 1, and the function of the gene is disrupted by targeting and cleaving at positions 257-277 of the sequence; or the gene encoding β-glucosidase has a nucleotide sequence set forth in SEQ ID NO: 3, and the function of the gene is disrupted by targeting and cleaving at positions 2607-2626 of the sequence.

9. Use of a regulator of a target gene for improving the efficiency of protein expression by *Trichoderma reesei;* wherein the regulator includes a down-regulator; wherein the down-regulator targets ORF4 protein or its encoding gene or targets endogenous glucosidase or its encoding gene.

10. The use according to claim 9, wherein the down-regulator includes an agent that targets and knocks out the target gene via CRISPR/Cas gene editing; or an agent that modifies the segment where the target gene is located with a deletion or an insertion via homologous recombination so that the target gene is knocked out; or an interfering molecule, including dsRNAs, antisense nucleic acids, small interfering RNAs or microRNAs, or constructs that can express or form the dsRNAs, antisense nucleic acids, small interfering RNAs, microRNAs, that target and inhibit or silence the target gene or transcript thereof.

11. A recombinant *Trichoderma reesei* strain or a spore, mycelium or protoplast thereof, in the genome of which a target gene is down-regulated; wherein the down-regulated target gene is selected from: a gene encoding ORF4 protein, a gene encoding endogenous β-glucosidase.

12. The *Trichoderma reesei* strain or a spore, mycelium or protoplast thereof according to claim 11, wherein in the *Trichoderma reesei* strain or the spore, mycelia or protoplast thereof, the target gene is inhibited, knocked out, silenced or mutated, or the expression or activity of the protein encoded by the target gene is inhibited; or the *Trichoderma reesei* strain or the spore, mycelia or protoplast thereof is transformed with a down-regulator of the target gene; preferably, the down-regulator of the target gene includes: an agent that inhibits or knocks out the target gene, or inhibits the expression or activity of the protein encoded by the target gene.

13. The *Trichoderma reesei* strain or a spore, mycelium or protoplast thereof according to claim 11, further comprising a gene encoding a heterologous protein; preferably, the gene encoding the heterologous protein is introduced into the *Trichoderma reesei* cell via an expression vector, or is inserted into the genome of the *Trichoderma reesei,* for example, into the cbhl site.

14. Use of the *Trichoderma reesei* strain or a spore, mycelium or protoplast thereof according to any of claims 11 to 13 for expressing a heterologous protein or an endogenous protein by *Trichoderma reesei.*

15. A method for recombinantly expressing a heterologous protein, comprising: inserting a gene encoding a heterologous protein into the genome of a recombinant *Trichoderma reesei* strain or a spore, mycelium or protoplast thereof according to any of claims 11 to 13; culturing the *Trichoderma reesei* strain or the spore, mycelium or protoplast thereof so that the heterologous protein is expressed; preferably, wherein the gene encoding the heterologous protein is inserted into the cbhl site in the genome of the *Trichoderma reesei* strain or the spore, mycelium or protoplast thereof.

16. A method for recombinantly expressing an endogenous protein of *Trichoderma reesei,* comprising: culturing the recombinant *Trichoderma reesei* or a spore, mycelium or protoplast thereof according to any of claims 11 to 13, so that the endogenous protein of *Trichoderma reesei* is recombinantly expressed.

17. A kit for protein expression, comprising: a recombinant *Trichoderma reesei* strain or a spore, mycelium or protoplast thereof according to any of claims 11 to 13; a down-regulator of the target gene, wherein the target gene is selected from: a gene encoding ORF4 protein, a gene encoding endogenous β-glucosidase.

18. The kit according to claim 14 or 16, wherein the endogenous protein includes: cellulase, hemicellulase, β-glucosidase, carbohydrate permease, and enzymes related to protein synthesis and secretion pathways; or, the heterologous protein includes non-cellulase secreted proteins; preferably, the heterologous protein includes: heterologous glucosidase, heterologous feruloyl esterase FEA, structural proteins, and functional proteins.
